# EUROPEAN PATENT SPECIFICATION

(11) **EP 2 776 584 B1**
(45) Date of publication and mention of the grant of the patent: **02.01.2019**
(21) Application number: 12795832.0
(22) Date of filing: 09.11.2012
(51) Int. Cl.: C12Q 1/68

(54) **METHOD AND PRIMERS FOR THE DETECTION OF MICROCYSTIN-PRODUCING TOXIC CYANOBACTERIA**
VERFAHREN UND PRIMER ZUR DETEKTION VON MIKROZYSTIN-PRODUZIERENDEN TOXISCHEN CYANOBAKTERIEN
PROCÉDÉS ET AMORCES POUR LA DÉTECTION DE CYANOBACTÉRIES TOXIQUES PRODUISANT DE LA MICROCYSTINE

(30) Priority: 09.11.2011 FI 20116109; 09.11.2011 US 201161557496 P
(43) Date of publication of application: 17.09.2014
(73) Proprietor: Turun yliopisto, 20014 Turun Yliopisto (FI)
(72) Inventor: SAVELA, Henna, FI-20100 Turku (FI); VEHNIÄINEN, Markus, FI-20660 Littoinen (FI)
(74) Representative: Seppo Laine Oy
(86) International application number: PCT/FI2012/051098
(87) International publication number: WO 2013/068654

(56) References cited:
- WO-A2-2004/104211
- BITTENCOURT-OLIVEIRA M C ET AL: "Toxic cyanobacteria in reservoirs in northeastern Brazil: detection using a molecular method", BRAZILIAN JOURNAL OF BIOLOGY = REVISTA BRASLEIRA DE BIOLOGIA BRAZIL,, vol. 70, no. 4, 1 November 2010 (2010-11-01), pages 1005-1010, XP009166807,
- SCHOBER ET AL: "Interlaboratory comparison of Taq Nuclease Assays for the quantification of the toxic cyanobacteria Microcystis sp", JOURNAL OF MICROBIOLOGICAL METHODS, ELSEVIER, AMSTERDAM, NL, vol. 69, no. 1, 28 March 2007 (2007-03-28) , pages 122-128, XP005928186, ISSN: 0167-7012, DOI: 10.1016/J.MIMET.2006.12.007
- NISHIZAWA T ET AL: "Genetic analysis of the peptide synthetase genes for a cyclic heptapeptide microcystin in Microcystis spp", JOURNAL OF BIOCHEMISTRY, JAPANESE BIOCHEMICAL SOCIETY / OUP, TOKYO; JP, vol. 126, no. 3, 1 September 1999 (1999-09-01), pages 520-529, XP002372135, ISSN: 0021-924X -& DATABASE EMBL [Online] 7 September 1999 (1999-09-07), "Microcystis aeruginosa mcyA, mcyB and mcyC genes, complete cds.", XP002691847, retrieved from EBI accession no. EMBL:AB019578 Database accession no. AB019578
- ROUHIAINEN L ET AL: "Genes coding for hepatotoxic heptapeptides (microcystins) in the cyanobacterium Anabaena strain 90", APPLIED AND ENVIRONMENTAL MICROBIOLOGY, AMERICAN SOCIETY FOR MICROBIOLOGY, US, vol. 70, no. 2, 1 February 2004 (2004-02-01), pages 686-692, XP002982238, ISSN: 0099-2240, DOI: 10.1128/AEM.70.2.686-692.2004 cited in the application -& DATABASE EMBL [Online] 10 June 2003 (2003-06-10), "Anabaena sp. 90 microcystin synthesis gene cluster", XP002692274, retrieved from EBI accession no. EM_STD:AJ536156 Database accession no. AJ536156
- CHRISTIANSEN G ET AL: "Microcystin biosynthesis in planktothrix: Genes, evolution, and manipulation", JOURNAL OF BACTERIOLOGY, AMERICAN SOCIETY FOR MICROBIOLOGY, WASHINGTON, DC; US, vol. 185, no. 2, 1 January 2003 (2003-01-01), pages 564-572, XP002982232, ISSN: 0021-9193, DOI: 10.1128/JB.185.2.564-572.2003 cited in the application -& DATABASE EMBL [Online] 5 November 2002 (2002-11-05), "Planktothrix agardhii microcystin synthesis gene cluster", XP002692275, retrieved from EBI accession no. EM_STD:AJ441056 Database accession no. AJ441056
- HENNA HAUTALA ET AL: "Quantitative PCR detection and improved sample preparation of microcystin-producing Anabaena, Microcystis and Planktothrix", ECOTOXICOLOGY AND ENVIRONMENTAL SAFETY, vol. 87, 1 January 2013 (2013-01-01), pages 49-56, XP055052179, ISSN: 0147-6513, DOI: 10.1016/j.ecoenv.2012.10.008
- SIVONEN KAARINA: "Emerging high throughput analyses of cyanobacterial toxins and toxic cyanobacteria", ADVANCES IN EXPERIMENTAL MEDICINE AND BIOLOGY, SPRINGER, US, vol. 619, 1 January 2008 (2008-01-01), pages 539-557, XP009166821, ISSN: 0065-2598, DOI: 10.1007/978-0-387-75865-7_24

## Description

### FIELD OF THE INVENTION

The present invention relates to the field of detection of microcystin-producing toxic cyanobacteria from environmental samples. Particularly, the present invention provides a polymerase chain reaction (PCR) based assay method for the detection of said toxic cyanobacteria. The present invention further provides materials such as primers, primer pairs and probes designed for the method of the invention.

### BACKGROUND OF THE INVENTION

In recent years, toxic cyanobacterial blooms have gained increasing amounts of attention by the scientific community, authorities and general public worldwide. Toxins produced by cyanobacterial mass occurrences have caused deaths of wildlife and livestock (see Stewart et al., 2008, for a review), and have been connected to human illness and death (Turner et al., 1990; Pilotto et al., 1997; Jochimsen et al., 1998). Microcystins, cyclic heptapeptide hepatotoxins that form a major cyanotoxin group with over 90 known structural variants (Welker and von Döhren, 2006) are produced by strains of *Anabaena, Microcystis, Planktothrix* and *Nostoc* (Sivonen and Jones, 1999), although isolated occurrences of microcystin-producing *Hapalosiphon* (Prinsep et al., 1992), *Phormidium* (Izaguirre et al., 2007) and *Fischerella* (Fiore et al., 2009) have been described. Of these eight genera, mostly *Anabaena, Microcystis* and *Planktothrix* cause freshwater hepatotoxic blooms. To better understand the dynamics of cyanobacterial blooms and mechanisms of toxin production, and predict the risks associated with these phenomena, easy-to-use detection methods for different microcystin variants and toxin-producing cyanobacteria are needed. Continuous low-level exposure to microcystins has been connected with increased risk of hepatic cancer (Yu, 1995; Ueno et al., 1996; Svircev et al., 2009), which further stresses the need for these methods.

Reliable identification of microcystin- and non-microcystin-producing cyanobacteria can be challenging. Mixed blooms of morphologically indistinct toxic and non-toxic strains of the same species are common (Vezie et al., 1998). The discovery of the *mcyS* gene cluster revealed a group of potential targets for PCR-based detection of microcystin producers.

Genes connected to the non-proteinogenic, rare amino acid residues in the toxin structure are especially well suited for this purpose (Mbedi et al., 2005). Many studies describe nucleic acid based methods to detect microcystin-producing cyanobacteria, and their use in attempt to evaluate the risk associated with toxic blooms (see Sivonen, 2008, for a review). For instance, WO2011003184 discloses primers designed for the detection of microcystin-producing cyanobacteria. The primers disclosed are complementary to the conserved region of the ss-ketoacyl synthase (KS) domain of the *mcyD* gene of Microcystis aeruginosa strain UTCC 299, or to the conserved region of the first dehydratase domain of the *mcyD* gene of Microcystis aeruginosa UTCC 300. In WO2006128230, another PCR-based method for detection of hepatotoxic cyanobacteria is disclosed. The target sequences for amplification are located in hepatotoxin-associated aminotransferase domain sequences derived from the *mcyE* gene of the microcystin synthetase gene complex and the *ndaF* gene of the nodularin synthetase gene complex.

However, few real-time quantitative PCRs capable of detecting more than one microcystin-producing genera have been described (Al-Tebrineh et al., 2011; Vaitomaa et al., 2003; Briand et al., 2008), and currently the majority of published qPCR methods concentrate on only one genus, most often *Microcystis* (Foulds et al., 2002; Kurmayer and Kutzenberger, 2003; Rinta-Kanto et al., 2005; Furukawa et al., 2006; Fortin et al., 2010).

Sample preparation and label technology choices are important in qPCR. Sample loss during multi-step DNA extraction processes combined with inefficient amplification caused by incomplete removal of PCR inhibitors can result in significant quantification error (Wilson, 1997). Addressing these problems would improve PCR reliability. Increased specificity, in turn, can be achieved with sequence-specific labelled probes (e.g., TaqMan), and unlike the more commonly used prompt fluorophores, lanthanide chelate labels allow detection by time-resolved fluorometry, leading to improved sensitivity due to lower background signals, since any autofluorescence decays during the time window between excitation and measurement (Soini and Lövgren, 1987)

The aim of this study was to develop a quantitative real-time PCR method for the detection of potentially microcystin-producing *Anabaena, Microcystis* and *Planktothrix.* To achieve reliable quantification, DNA extraction and simple cell lysis were compared in terms of qPCR performance and template yield. The developed qPCR assay was applied to environmental sample analysis, and the correlation between gene copy numbers and microcystin concentrations examined.

### BRIEF DESCRIPTION OF THE DRAWINGS

**Figure 1****.** Standard curves for the *mcyB* qPCR assay. Base 10 log *mcyB* copy numbers are plotted against their respective treshold cycles (Cₜ) for a) *Anabaena,* b) *Microcystis* and c) *Planktothrix.* A range of 10¹-10⁷ copies of the *mcyB* target sequence could be detected for each target genera. Amplification efficiences were similar (92.7-94.2 %) for all targets. Variation in Cₜ within the four replicate reactions was very low, standard deviations are shown as error bars mostly hidden inside the symbols.

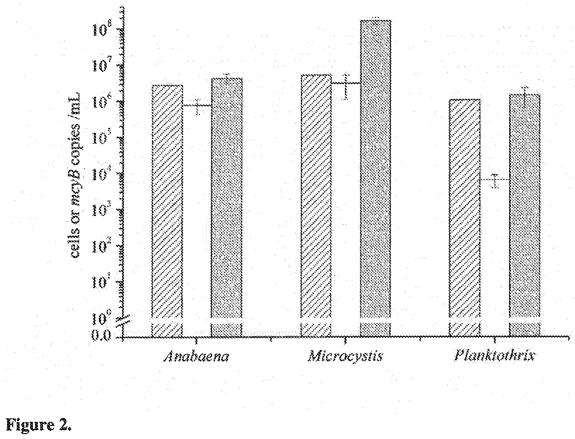

**Figure 2****.** Comparison of two qPCR sample preparation methods. Microscopically determined cell amounts (patterned columns) were compared to detected *mcyB* gene copies in extracted genomic DNA (white columns) and in filtered and disrupted cells (grey columns). Comparisons were made for microcystin-producing *Microcystis aeruginosa* NIVA-CYA 140, *Anabaena cf. flos-aquae* NIVA-CYA 267/4 and *Planktothrix agardhii* NIVA-CYA 299. Filtering and cell lysis yielded consistently higher copy number compared to DNA extracted from the same amount of cells. Error bars indicate both inter- and intra-sample copy number variation.
**Figure 3****.** The development of *Planktothrix mcyB* gene copy numbers (■, solid line), *Planktothrix* cell numbers (▲, dotted line), and total microcystin concentrations determined by LC-MS (□, dashed line) in Hauninen reservoir (Raisio, Finland) during April-June 2008. Cell numbers are based on microscopic counting of 100 µm filament units and an estimate of 30 cells per one such unit. A clear positive correlation between *mcyB* copy numbers and total microcystin concentration (dmMC-RR and dmMC-LR) was observed. No potentially toxic *Microcystis* or *Anabaena* was detected.
**Figure 4****.** Target sequences in *mcyB* gene of *Anabaena* sp., *Microcystis aeruginosa* and *Planktothrix agardhii,* and primers and probes used in the Example.

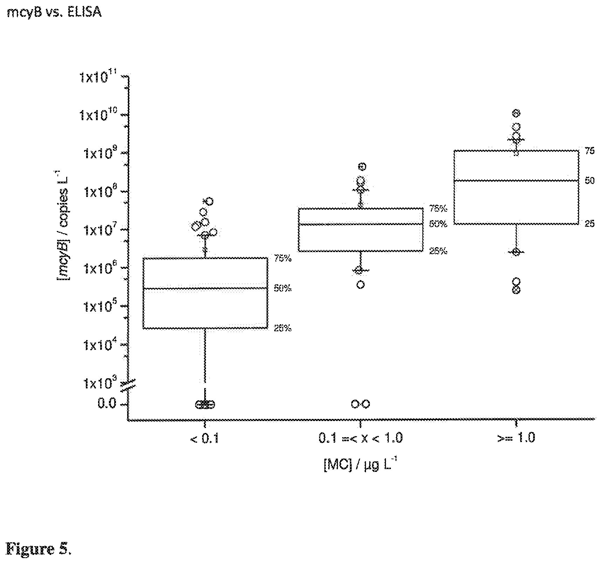

**Figure 5****.** Correlation of total gene copy number of the *mcyB* gene to microcystin concentrations [MC] in an environmental aquatic sample measured by ELISA.

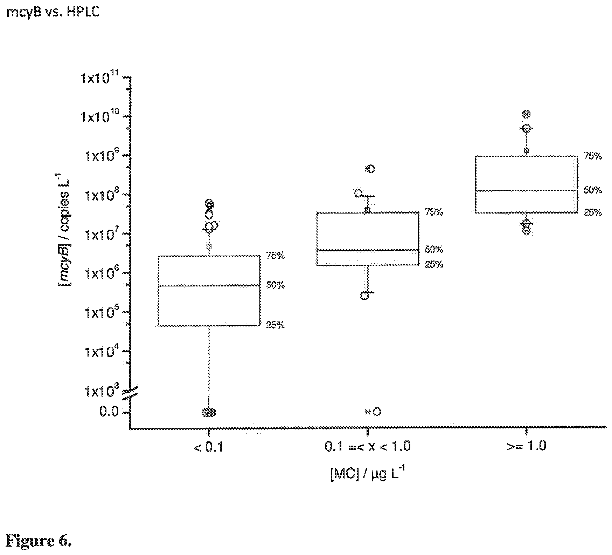

**Figure 6****.** Correlation of total gene copy number of the *mcyB* gene to microcystin concentrations [MC] in an environmental aquatic sample measured by HPLC.

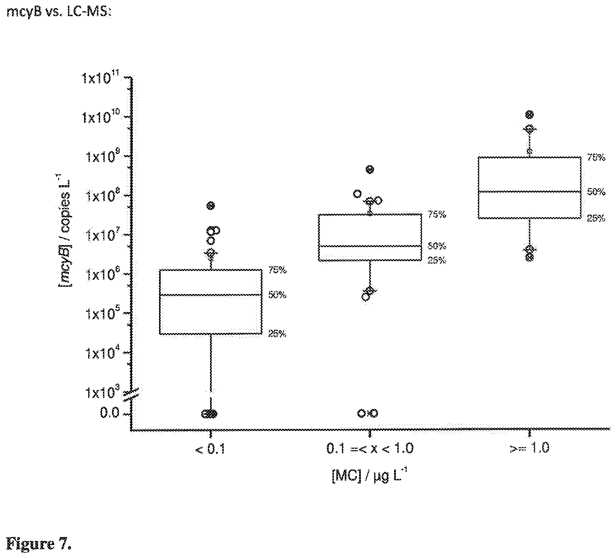

**Figure 7****.** Correlation of total gene copy number of the *mcyB* gene to microcystin concentrations [MC] in an environmental aquatic sample measured by LC-MS.

### DETAILED DESCRIPTION OF THE INVENTION

The present invention is defined by the appended claims.

The present invention is directed to a method for detecting the presence of microcystin-producing toxic cyanobacteria in a sample comprising the steps of:
a) performing lysis of the cells in said sample;
b) contacting nucleic acid obtained from the lysed cells in a polymerase chain reaction mix with primers hybridizing with the nucleic acid sequence of SEQ ID N° 1, SEQ ID N° 2 and SEQ ID N° 3 in the *mcyB* gene present in *Microcystis aeruginosa, Planktothrix agardhii* and *Anabaena sp*., wherein said primers amplify at least part of the target sequence in the *mcyB* gene
c) performing a polymerase chain reaction with a reaction mix obtained from step b) so that the sequences of said *mcyB* gene are specifically amplified, if said sequences are present in the sample; and
d) detecting the presence of amplified nucleid acid sequences, wherein the presence of amplified *mcyB* gene sequence is indicative of the presence of microcystin-producing toxic cyanobacteria in the sample.

The samples prepared for the method of the invention comprising cyanobacterial material are preferably environmental samples, such as water samples which can be seawater samples or freshwater samples or other suitable environmental samples.
While the method is mainly directed to the detection of toxic cyanobacteria, such as *Microcystis aeruginosa, Planktothrix agardhii* and *Anabaena sp*., it can also be used for the quantification of the microcystin-producing toxic cyanobacteria in said samples. This can be done by performing quantitative-PCR (qPCR) on the extracted DNA using the primers of the present invention, and determining the gene copy number of the *mcyB* gene. Further, the toxic microcystin concentration in the sample may also be monitored by correlating said gene copy number obtained by qPCR to a corresponding microcystin concentration.

In step a), the nucleic acid which is used as template in the PCR of step b) can be extracted and purified from the lysed cells by conventional methods. However, the suspension obtained from the lysed cells is preferably used directly as PCR template. Accordingly, in one embodiment of the invention an undiluted water sample suspected to comprise toxic cyanobacteria can be first filtered. Then filtered cells are suspended in sterile deionized water and cell lysis is carried out by heat treatment. The obtained suspension and nucleic acid therein can be used as such for the reaction mix of step b).

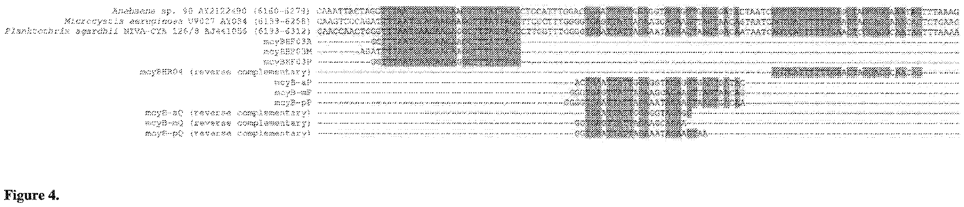

In step b), said primers amplify at least part of the target sequence in the *mcyB* gene as set forth in SEQ ID NO:1 corresponding to positions 6147-6251 of *Microcystis aeruginosa* genome described in Figure 4, or as set forth in SEQ ID NO:2 corresponding to positions 6203-6305 of *Planktothrix agardhii* genome described in Figure 4, or as set forth in SEQ ID NO:3 corresponding to positions 6170-6272 of *Anabaena sp.* described in Figure 4.

In embodiments of the invention, the amplicon, i.e. the target sequence, which is amplified in step b), comprises at least 20, preferably at least 50, more preferably at least 80, and most preferably 103 or 105 consecutive nucleotides of the target sequence as defined by SEQ ID NO:1, SEQ ID NO:2 or SEQ ID NO:3.

Preferably, the primers used in the method comprise or consist of one of the following sequences:
5'-GCTTTAATCCACAAGAAGCTTTATTAGC-3' (SEQ ID NO:4)
5'-AGATTTTAATCCACAAGAAGCTTTATTAGC-3' (SEQ ID NO:5)
5'-GGTTTAATCAACAAGAGGCTTTATTAGC-3' (SEQ ID NO:6)
5'-CTGTTGCCTCCTAGTTCAAAAAATGACT-3' (SEQ ID NO:7)
In one of the most preferred embodiment of the present invention, said method is performed as a real-time polymerase chain reaction for which several commercial kits are available. For use in real-time polymerase chain reaction, the present invention also provides oligonucleotide probes specifically hybridizing with the nucleic acid sequence of *mcyB* gene present in *Microcystis aeruginosa, Planktothrix agardhii* and *Anabaena sp*., wherein the probes have the sequence:
5'-ACTGAATTATTGGAGGTAGAGGTGAGTGATAC-3' (SEQ ID NO:8)
5'-CCTCTACCTCCAATAATTCA-3' (SEQ ID NO:9)
5'- GGGTGAGTTATTAGAAGCAGAAGTTAGTAACAG-3' (SEQ ID NO:10)
5'-TTCTGCTTCTAATAACTCACC-3' (SEQ ID NO:11)
5'-GGGGTGAATTATTAGAAATAGAAGTAAGTGACAA-3' (SEQ ID NO:12)
5'-TTACTTCTATTTCTAATAATTCACC-3' (SEQ ID NO:13)

Also disclosed are primers comprising or consisting of any of the sequences as set forth in SEQ ID NOS:4-7. Preferably, the primer has 28-40 nucleotides. More preferably, the primer comprises less than 30, 35 or 40 nucleotides.

Further, are provided probes comprising or consisting of any of the sequences as set forth in SEQ ID NOS:8-13. Preferably, the probe has less than 40 nucleotides.

The oligonucleotide primers and probes are short sequences of nucleotides (such as RNA or DNA, preferably DNA), typically with twenty-five to thirty or fewer bases. However, automated synthesizers allow the synthesis of oligonucleotides up to 160 to 200 bases and the present oligonucleotides may be elongated to add, e.g., a restriction enzyme cleavage site, to the oligonucleotide. The typical length of the primers is preferably 26-32, more preferably 28-30 nucleotides.

The present invention also provides kits for the detection of the presence of microcystin-producing toxic cyanobacteria. The kit may comprise primers and probes as defined above. Such primers and probes are described above and in the Examples below.

Preferably, said kit comprises means for a real-time polymerase chain reaction, such as labelled probes, polymerase enzymes, buffers and nucleotides.

It is well-known in the art that the sequences of same gene vary somewhat in strains of a microbial species and, thus, the sequence of the *mcyB* gene present in *Microcystis aeruginosa, Planktothrix agardhii* and *Anabaena sp.* is not 100% identical in all strains of the species. However, it is clear from the description herein, particularly from the Example below, that a person skilled in the art would recognize the sequence of the *mcyB* gene in any related strain based on the similarity and homology of these gene sequences.

Herein the term "specifically hybridizing" means complementary hybridization between an oligonucleotide and a target sequence. The term "specifically" refers to the specificity shown by the complementary hybridization, which allows for minor mismatches between the oligonucleotide and the sequence that may not jeopardize the annealing for detection of hybridization signals.

The present invention is further described in the following example, which is not intended to limit the scope of the invention.

### EXPERIMENTAL SECTION

### Materials and Methods

### 1.1. Cyanobacterial strains and environmental samples

Cyanobacterial strains used in this study are listed in Table 1. Strains were purchased from the Pasteur Culture Collection (PCC, Paris, France) and the Norwegian Institute for Water Research Cyanobacterial Culture Collection (NIVA, Oslo, Norway) and maintained as recommended by the providers. All NIVA strains were cultured in Z8 (Staub, 1961, modified NIVA 1972, 1976). Strains from PCC were cultured in either BG 11 (Sigma), nitrate omitted BG11₀, or BG11₀ with added NaNO₃ and NaHCO₃ using formulations described by PCC. Three additional strains from other sources were also maintained. *Microcystis aeruginosa* NIES-107 (National Institute of Environmental Studies, Tsukuba, Japan) was cultured in Z8. *Anabaena lapponica* strain 966 (Finnish Environment Institute, Dr. Jarkko Rapala) as well as *Anabaena* sp. 90 (University of Helsinki, Prof. Kaarina Sivonen) were cultured in modified Z8 with no added nitrogen. All cultures were maintained at 23 °C using PowerGlo 20 W aquarium bulbs (Hagen, Japan) as the light source. Cyanobacterial cells were microscopically counted from 2.5 week old cultures of NIVA-CYA 267/4, NIVA-CYA 140 and NIVA-CYA 299. Samples were diluted 1:100-1:200 in deionized water and preserved in Lugol's iodine. After a 16 h sedimentation of 10 ml sample aliquots counting was carried out on a Nikon TE-200 inverted microscope (Nikon, Japan) with 10x and 40x objectives. Cells from enumerated cultures were harvested for *mcyB* quantification. Environmental samples were collected from Hauninen reservoir, Raisio, Finland during April-June 2008 (11 samples) and from freshwater lakes at Åland Islands during July 2008 (13 samples). All samples, including extracted DNA and other PCR templates, were kept frozen at -20 °C until further analysis. Culture and environmental samples were either frozen fresh or freeze-dried.

### 1.2. Extraction of microcystins

Samples of eight to ten mg freeze-dried cyanobacterial material or freeze-dried GF/C fiberglass filters (Ø 25 mm; Whatman, Maidstone, UK) containing filtered cyanobacterial material were extracted with 1.2 ml of 75% methanol (HPLC grade; Rathburn, Walkerburn, UK). Extracts were treated for 15 min in a bath ultrasonicator (Bandelin Sonorex RK 156, Berlin, Germany) and additionally for 1 min with a probe sonicator (Bandelin Sonopuls HD 2070 with 3 mm microtip, 30% pulse, 30% energy). After centrifugation at 10,000 x g for 10 minutes, the supernatants were divided into aliquots and evaporated to dryness with argon at 40 °C. Extracts intended for microcystin analysis by HPLC-DAD and LC-ESI-MS-MS were resuspended in 75% methanol while those aimed for ELISA in water.

### 1.3. HPLC with diode-array UV detection (HPLC-DAD)

Samples were analysed using an Agilent (Waldbronn, Germany) 1100 series HPLC system consisting of a degasser, a quaternary pump, a thermostatted column compartment at 40 °C and a diode-array detector operated at 200-300 nm (quantification at 238 nm). The stationary phase was either a Purospher STAR column, 3 µm particles, 55 mm × 4 mm from Merck (Darmstadt, Germany) or an Ascentis RP-Amide column, 3 µm particles, 100 mm × 4.6 mm I.D. from Supelco (Bellefonte, PA, USA). The mobile phase consisted of acetonitrile (HPLC S grade, Rathburn, Walkerburn, UK) (solvent B) - Milli-Q ultrapure water (Millipore, Molsheim, France) (solvent A) both containing 0.05% trifluoroacetic acid (TFA; Fluka, Buchs, Switzerland) with the following linear gradient programme: 0 min 25% B, 7 min 70% B, 10 min 70% B, 10.1 min 25% B; stop time 15 min; flow-rate 1 ml/min. Injection volumes were 10 µl. Additionally, the samples were analysed on a Merck Purospher STAR RP-18e column, 55 mm × 4 mm I.D. with 3 µm particles (Spoof and Meriluoto, 2005). Individual laboratory-purified microcystins and following reference samples were used for the identification of microcystins: a) extract of *Microcystis aeruginosa* PCC 7820 and b) extract of *Microcystis aeruginosa* NIES-107 as described in (Spoof et al., 2003).

### 1.4. LC-MS and LC-MS-MS

LC-MS experiments were performed on an Agilent 1100 Series HPLC system coupled to a Waters Micromass (Manchester, UK) Quattro Micro triple-quadrupole mass spectrometer equipped with an electrospray interface. Toxins were quantified on a Merck Purospher STAR RP-18 endcapped column (30 mm × 4 mm, 3 µm particles). The mobile phase consisted of a gradient of 0.1% aqueous formic acid (solvent A) and acetonitrile (solvent B) with the following linear gradient program: 25% B to 70% B over 10 min, then to 90% B over 2 min, where it was held for 1 min. Injection interval was 16 min, injection volume 10 µl, flow rate 0.5 ml/min, and column oven temperature 40 °C. Capillary voltage was set at 3.8 kV and cone voltage at 40 V (dmMC-RR and MC-RR) or 75 V (rest of the microcystins and nodularin). Desolvation gas (nitrogen) temperature and flow rate were set at 300 °C and 650 L/h, respectively. Ion source temperature was set at 150 °C. Ions were detected in the positive electrospray ionization mode. The monitored signals in the selected ion recording (SIR) mode were *m*/*z* [dmMC-RR+2H]²⁺ 512.8, [MC-RR+2H]²⁺ 519.8, [MC-LF+H]⁺ 986.5 and [MC-LF+Na]⁺ 1008.5, [dmMC-LR+H]⁺ 986.5, [MC-LR+H]⁺ 995.5, [MC-LY+H]⁺ 1002.5, [MC-LW+H]⁺ 1025.5 and [MC-LW+Na]⁺ 1047.5, [MC-YR+H]⁺ 1045.5, [dmNod+H]⁺ 811.5 and [Nod+H]⁺ 825.5. Data acquisition was done with Masslynx v. 4.0 software (Micromass). LC-MS-MS experiments were carried out on an Agilent 1200 Rapid Resolution (RR) LC coupled to a Bruker Daltonics HCT Ultra ion trap mass spectrometer (Bremen, Germany) with electrospray ion (ESI) source. The 1200 RR LC system included a binary pump, a vacuum degasser, a SL autosampler, and a thermostatted column compartment. The ion trap was operated in the positive electrospray ion mode. Ion source parameters were set as follows: dry temperature 350 °C, nebulizer pressure 40 psi, dry gas flow 10.0 L/min, capillary voltage 4.0 kV. An MS scan range from 500 to 1200 m/z with the Smart Parameter Setting (SPS) function was employed. The ICC target was set to 300 000 with a maximum accumulation time of 100 ms. Abundant MS-MS fragmentation was assisted by the SmartFrag setting. Separation of toxins was achieved on Ascentis C₁₈, 50 mm × 3 mm I.D. column with 3 µm particles (Supelco) at 40°C. Injection volumes were 5 µl. The mobile phase consisted of water-acetonitrile-formic acid (99:1:0.1; solvent A) and acetonitrile-formic acid (100:0.1; solvent B) with the following linear gradient programme: 0 min 25% B, 5 min 70% B, 6 min 70% B, 6.1 min 25% B; stop time 10 min: flow-rate 0.5 ml/min. Primary monitored signals were the same as with the Quattro micro instrument but mass spectra acquired with the HCT Ultra instrument allowed for identification of additional toxins based on fragmentation patterns. Data acquisition was done with Bruker Compass 1.3 software.

### 1.5. ELISA

Samples were analysed with the Quantiplate Microcystin kit (Envirologix, Portland, ME, USA) using the protocol provided by the manufacturer. Extracts were diluted with water according to HPLC results to adjust the microcystin concentrations to the working range of the assay. Two different dilutions were made of some samples.

### 1.6. PCR sample preparation

Two methods were used to prepare samples for PCR analysis. Genomic DNA was extracted from freeze-dried cells of cultured strains or freeze-dried Åland Islands environmental samples using the NucleoSpin Plant II DNA extraction kit (Macherey-Nagel, Düren, Germany) according to the manufacturer's instructions. DNA concentration and quality were determined spectrophotometrically (ND-1000, NanoDrop Technologies, Wilmington, DE, USA). Filtering and cell lysis was performed as follows: 10 ml of harvested cell culture was suspended in 90 ml of sterile deionized water, and vacuum filtered on a fiberglass filter (Ø 47 mm GF/C, Whatman). In the case of Hauninen reservoir samples, 50 ml of undiluted water was filtered. Pieces of each filter were cut out and suspended in 100 µl of sterile deionized water. Cell lysis was carried out at 80 °C for 5 min. The suspension was thereafter used directly as PCR template. Filter piece dimensions were recorded for quantification purposes. Microscopically counted culture samples were treated using both methods described above, with a few modifications: cells were harvested as aliquots of both 5 ml and 10 ml, and subjected either to filtration or DNA extraction. Sample preparation was carried out on freshly harvested cells. Cells were collected for DNA extraction by centrifugation (4 °C, 3220 g, 20 min, Eppendorf 5810R, Hamburg, Germany).

### 1.7. Primers and probes

*mcyB* sequences (Nishizawa et al., 1999; Tillett et al., 2000; Christiansen et al., 2003; Rouhiainen et al., 2004; Kaneko et al., 2007) were retrieved from the GenBank Nucleotide database, and primers and probes were designed based on the sequence alignment. Oligonucleotides were manufactured by Thermo Scientific (Ulm, Germany) and biomers.net (Ulm, Germany) (Table 2). Probes were synthesized with 5'-C6-aminolinkers and 3'-phosphate groups. All detection probes were labeled at the 5' end aminolinker with an organic Tb³⁺ chelate (2,2',2",2'"-{{6,6'-{4"-[2-(4-Isothiocyanatophenyl)ethyl]pyratzole-1",3"-diyl}bis(pyridine)-2,2'-diyl}bis(methylenenitrilo)}tetrakis(acetato)}terbium(III)) as described previously (Nurmi et al., 2002). All quencher probes had a BHQ1 (Black hole quencher® 1) molecule conjugated at their 3'-ends by the oligonucleotide manufacturer. Primer specificity was tested on the cyanobacterial strains listed in Table 1. Individual PCR reactions contained 5 nmol of dNTPs (Finnzymes, Espoo, Finland), 2.67 pmol of each forward primer and 8 pmol of the reverse primer, 0.5 units of DyNAzyme II HotStart DNA polymerase (Finnzymes), 1X DyNAzyme II HotStart buffer and 1 µl of template, either 1 ng of genomic DNA or 1 µl of suspension containing lysed cells. The reaction volume, 20 µl, was filled with sterile Milli-Q water. Thermocycling was performed using a PTC-200 Thermal Cycler (MJ Research, Watertown, MA, USA), starting with 10 min at 95 °C, followed by 35 cycles of 30 s at 95 °C, 30 s at 58 °C and 1 min at 72 °C, and ending with 10 min at 72 °C. The PCR products were analyzed on a 2 % agarose gel stained with ethidium bromide (0.5 ng L⁻¹).

### 1.8. qPCR

The principle of the real-time quantitative PCR technique has been described in detail elsewhere (Nurmi et al., 2002). Briefly, amplification product accumulation is monitored by measuring the long-life fluorescence obtained from chelate moieties freed from detection probes by the 5'→3' exonuclease activity of the DNA polymerase. Background fluorescence is kept at minimum with the help of quencher probes. Detection probe specificity was confirmed using qPCR and purified genomic DNA from all cultured strains. Each qPCR reaction contained 4 nmol of dNTPs, 2 pmol of each forward primer and 6 pmol of the reverse primer, 0.2 units of DyNAzyme II HotStart DNA polymerase, 1X DyNAzyme II HotStart buffer, 0.25 pmol detection probe (either mcyB-mP, mcyB-pP or mcyB-aP), 2.5 pmol of corresponding quencher probe (mcyB-mQ or mcyB-pQ, for mcyB-aQ the amount 3.0 pmol was used) and 1 ng of template DNA. The reactions were filled with sterile Milli-Q water to 20 µl. All reactions were run on ThermoFast 96 Robotic PCR Plates (Abgene, Surrey, UK) with Applied Biosystems Optical Caps (Foster City, CA, USA). The qPCR runs were started with 5 min at 95 °C, followed by 8 cycles of 30 s at 95 °C and 1 min at 62 °C. After cycle 8 the temperature was lowered to 35 °C for 15 s for fluorescence measurement. Measurements were repeated every second cycle until after cycle 40, and were carried out with Victor² 1420 Multilabel Counter (PerkinElmer Life Sciences Wallac, Turku, Finland) using the manufacturer's standard protocol for time-resolved measurement of Tb fluorescence.

### 1.9. Standard curve development

Double-stranded DNA standards were produced by PCR from *Anabaena* sp. 90, PCC 7806 (*Microcystis*) and NIVA-CYA 299 (*Planktothrix*) as described above. Purification of *mcyB* amplification products was performed using the Qiaquick PCR Purification kit (Qiagen, Hilden, Germany), and subsequent quantification using the QuantIt PicoGreen kit (Invitrogen, Eugene, OR, USA), both according to manufacturer's instructions. Molar concentrations of the purified amplification products were calculated, and a series of 10-fold dilutions (n=7) of each genus-specific standard was prepared. The standards were kept at - 20 °C until use. qPCRs were run as described above. Treshold cycles (Cₜ) were plotted against log *mcyB* copy numbers, and amplification efficiences (E) were calculated from the slope of the linear regression (E=10^{-1/slope}-1, where the value 1.00 corresponds to an efficiency of 100%).

### 1.10. Environmental and culture sample analysis by qPCR

Series of 10-fold dilutions (n=4) of templates prepared from microscopically counted culture samples were prepared. The aims were to compare qPCR quantification and cell densities and to assess amplification efficiency and possible PCR inhibition. All qPCRs were performed as described above, except for the increased template amount, 4 ng of DNA or 4 µl of heat-treated sample. Culture samples were analyzed in four replicate reactions, undiluted environmental samples in triplicate. Environmental samples that yielded a *mcyB* negative result were tested for PCR inhibition by adding 1 ng of *Anabaena* sp. 90 genomic DNA to each reaction and visualizing the PCR products on ethidium bromide stained agarose gels as described above.

### Results

### 2.1. Primer and probe specificity

Specificity of the *mcyB* primers and detection probes was tested on extracted DNA and heat-treated cells of 30 cyanobacterial strains. Probe specificities, microcystins and other toxins produced by the strains are listed in Table 1. No false negatives were observed. No amplification products were observed from non-microcystin-producing strains, except for nodularin-producing *Nodularia harveyana* PCC 7804. However, the *Nodularia* amplification product was not detected by any of the detection probes in qPCR. Thus, all genus-specific detection probes performed with 100 % specificity and sensitivity. Each of the three possible primer combinations could be used to amplify the target *mcyB* sequence from all microcystin-producing strains independent of genus, but amplification efficiences varied from strain to strain (data not shown). When the three forward primers were used together, efficient amplification was achieved.

### 2.2. Standard curves and amplification efficiency

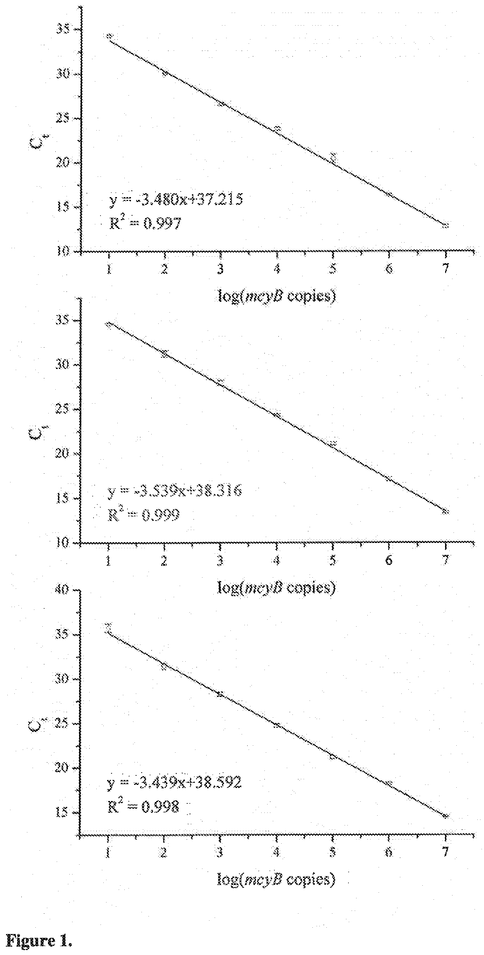

Sensitivities and amplification efficiencies were determined using standards prepared from purified PCR products. The analytical sensitivity was 10 *mcyB* copies per reaction and log-linear range 10 to 10⁷ copies of *mcyB* for all three target genera (Figure 1). Amplification efficiences and correlation coefficients derived from the standard curves were 93.8% (r²=0.997), 91.7% (r²=0.999) and 95.3% (r²=0.998) for *Anabaena, Microcystis* and *Planktothrix,* respectively.

### 2.3. Comparison of sample preparation methods

To compare the two sample preparation methods, PCR templates were prepared from culture samples of known cell density. The amplification efficiences obtained from templates prepared by filtration and heat-treatment were either higher or equal to those of corresponding purified DNA templates (89-100 %, average r²=0,9993±0,0008 and 83-97 %, average r²=0 9976±0,0018, respectively). The quality of extracted DNA varied, A₂₆₀/A₂₈₀ ratios ranged from 1.7 to 2.0. Heat-treated samples were too dilute to be examined spectrophotometrically. Filtration and heat-treatment of samples yielded 4-330 times the *mcyB* copy numbers of the corresponding extracted DNA (Figure 2). The greatest difference was observed in NIVA-CYA 299, a *Planktothrix agardhii* strain, where the detected copy numbers of filtered samples were only 1,5 times the cell counts, but 330 times the copy numbers in corresponding DNA extracted samples. For *Anabaena,* the copy numbers in DNA and cell lysates were of the same magnitude as cell counts. For *Microcystis,* the same was true for cell counts and copy numbers from extracted DNA, whereas filtration and heat-disruption of cells yielded copy numbers roughly 30 times the cell amount.

### 2.4. qPCR analysis of environmental samples

A total of 24 environmental samples were analyzed. The majority of Åland Islands lake samples (9/13) contained *mcyB* gene copies originating from either *Microcystis* only, or *Microcystis* together with *Anabaena* or *Planktothrix* (Table 3). Microcystins were present in all samples which in qPCR yielded a positive result for *mcyB*. However, a linear correlation between total copy numbers and toxin concentrations could not be established. Four samples did not contain target genera *mcyB* copies, nor showed evidence of PCR inhibition that could have caused false negative results. ELISA results were positive for all *mcyB* negative Åland Islands samples, whereas HPLC-DAD results for the same samples were all negative and LC-MS-MS analyses resulted in concentrations ranging from 0 to 0.67 µg microcystins per g of sample (dry weight).

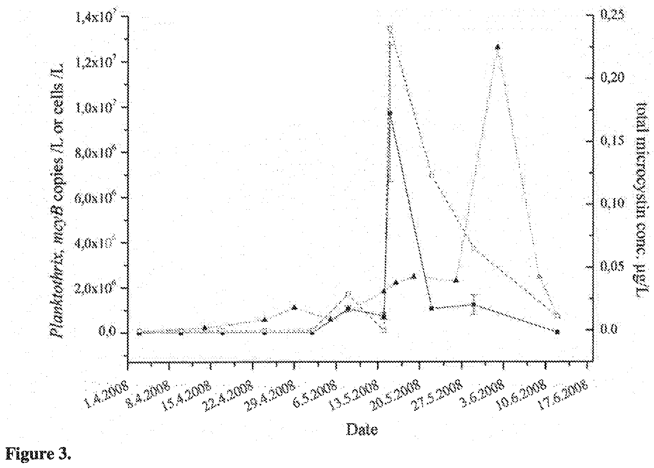

Five out of eleven Hauninen reservoir samples were positive for *mcyB.* In agreement with microscopical examinations, the population was dominated by *Planktothrix,* and no potentially toxic *Microcystis* or *Anabaena* were detected. PCR inhibition was not observed on visual examination of amplification products obtained from spiked samples. A clear positive correlation between *Planktothrix mcyB* copy numbers and total microcystin concentrations was found (Figure 3). Filament amounts increased gradually from April 3rd onward and reached a peak on May 29^{th}, after which a rapid decrease was observed. The simultaneous peaking of total microcystin concentration and *Planktothrix*-*specific mcyB* copy numbers did not coincide with the highest observed population density of *Planktothrix.* Two main microcystin variants were present in toxin-containing samples, dmMC-RR and dmMC-LR.

### Discussion

### 3.1. mcyB qPCR and the genetic background

Identification of a suitable target for the detection of several toxic genera is crucial. In the qPCR assay described here the target sequence is located within the second thiolation motif of *mcyB*, in a peptidyl carrier protein domain coding sequence, the domain that serves to transfer the growing polypeptide chain from McyB to McyC for further elongation (Nishizawa et al., 1999; Tillett et al., 2000) To our knowledge, this is the first time it has been used as a target for qPCR detection. Unlike the *mcyB* first adenylation domain targeted in many studies (e.g. (Mbedi et al., 2005; Kurmayer and Kutzenberger, 2003; Nonnemann and Zimba, 2002; Mikalsen et al., 2003), the second thiolation motif is not likely to be affected by microcystin structure variation. Simultaneous detection of several potentially toxic genera, independent on toxin profiles, is therefore possible. The wide range of potential hepatotoxin producers deteted was demonstrated by the amplification of *ndA,* homologous to *mcyB*, from the nodularin-producing *Nodularia harveyana* PCC 7804. The second condensation, adenylation and thiolation motifs of *mcyB* have their counterparts in the *ndaA* gene (Moffitt and Neilan, 2004), and the amplicon in *Nodularia* shares 80%, 75% and 76% sequence identity with the corresponding amplicons in *Anabaena, Microcystis* and *Planktothrix,* respectively. However, since the detection probes were shown to hybridize only with their intended targets, *mcyB* qPCR specificity was not compromised by the amplification *of ndaA.*

The majority of the existing quantitative *mcyB* qPCR assays are based on a few probes (if TaqMan detection chemistry is used) and primer pairs (Kurmayer and Kutzenberger, 2003; Nonnemann and Zimba, 2002; Kaebernick et al., 2002), which have a single target genus. With the new primers and genus-specific probes designed to detect a different part of the same gene, quantitative *mcyB* detection is expanded to include also *Anabaena* and *Planktothrix,* and an addition of a new target to the repertoir assays designed to detect more than one microcystin-producing genera is made. While generel primers provide information about the abundance of all toxic species (Al-Tebrineh et al., 2011), genus-specific detection has the added benefit of population dynamics monitoring. The sensitive label technology (Nurmi et al., 2002) used provides the assay with good sensitivity and a broad quantification range. With the detection limit of 10 *mcyB* copies per reaction and a range of seven orders of magnitude the current assay is very similar to previously published methods (Al-Tebrineh et al., 2011; Vaitomaa et al., 2003; Foulds et al., 2002; Kurmayer and Kutzenberger, 2003; Rinta-Kanto et al., 2005; Furukawa et al., 2006).

### 3.2. Sample preparation

Differences between the amplification efficiences of qPCR standards and the samples can have a substantial effect on the quantification result (Wilson, 1997). The same error source exists also between samples prepared by different methods, and any comparisons need to be made carefully. In this study, the theoretical copy number differences between sample types caused by variation in the observed amplification efficiences remained in the range of 3-5 fold. This cannot explain the difference of 1-2 orders of magnitude in detected copy numbers (for *Microcystis* and *Planktothrix*), showing that there is an actual significant difference between the amounts of available template per cell that simple lysis of the cyanobacterial cells and extraction and purification of genomic DNA produce. A number of reasons could cause such results, likely sample material loss and inefficient cell lysis during DNA extraction. It is also possible that discrepancies between gene copy numbers and cell counts is to some degree caused by errors in microscopic quantification, although an enumeration performed by an expert will make it considerably less probable.

Based on our observations, if one gene copy per cell was assumed, in a heat-treated sample *Microcystis mcyB* numbers would result in overestimation of cell concentration. However, under the same assumption, quantification of *mcyB* from purified genomic would DNA lead to underestimation of cell concentration, especially in the case of *Planktothrix.* Quantitative PCR results leading to cell number overestimation have been observed before for *Anabaena* and *Microcystis* (Vaitomaa et al., 2003), and studies have supported cyanobacterial cells hosting multiple genomes (Labarre et al., 1989; Becker et al., 2002). If this were the case, it would explain overestimations, but at the same time make underestimations more severe. Therefore we chose to use filtration and heat-treatment in the analysis of environmental samples whenever possible, and to compare copy numbers directly to toxin concentrations, avoiding the problematic translation of gene copies into cell numbers.

Similar results to the comparison made here have been reported for qualitative analyses before - alternative sample preparation methods have yielded templates comparable to purified DNA (Hisbergues et al., 2003; Ouahid et al., 2005). Quantitative performance assessments of different sample preparation methods have found phenol-chloroform extraction of DNA to yield higher copy numbers and therefore also higher estimations of cell concentrations than a commercial reagent kit for DNA extraction (Schober and Kurmayer, 2006). Rasmussen et al., 2008 found that ultrasonication and microwave treatment in detergent solutions performed as well as DNA extraction by a commercial reagent kit. Our observations agree with previously published results which indicate that cell lysis and DNA release without purification is a valid method for sample preparation preceding detection and quantification of *mcy* gene targets.

### 3.3. Environmental samples

The cyanobacterial population in Hauninen reservoir was followed by quantifying *mcyB* copy numbers and total microcystin concentrations during spring and early summer 2008. Although in theory the presence of microcystin synthetase genes only reveals the potential for toxin production, the positive correlation between *Planktothrix mcyB* copies and toxin concentrations was evident. Since neither of the two other targeted toxin producers were detected, *Planktothrix* was assumed the sole microcystin producer genus of the reservoir. This conclusion was supported by the microcystin variants found in the samples, MC-dmLR and MC-dmRR, which are typical for *Planktothrix* (Fastner et al., 1999). Filament counts had no clear connection to *mcyB* copy numbers or toxin concentrations, which indicated that the *Planktothrix* population in Hauninen reservoir was a mix of toxic and non-toxic strains, their relative proportions varying widely during the monitoring period. Inactive *mcy-*genotypes in *Planktothrix* have been reported, and they appear to be fairly common (Kurmayer et al., 2004; Ostermaier and Kurmayer, 2009). The presence of such genotypes in the Hauninen reservoir could not be inferenced based on the observed toxin concentrations and gene copy numbers, and was therefore assumed negligible.

In contrast to Hauninen reservoir, single samples collected from separate freshwater lakes on Åland Islands did not show clear correlation between microcystin concentrations and *mcyB* copy numbers, although no false positive qualitative *mcyB* results (i.e. gene copies detected but no microcystins present in the sample) were observed. Environmental factors can introduce variation into *mcy* gene expression patterns and toxin production (Kaebernick et al., 2000; Sevilla et al., 2008), and therefore differeces in the lake environments most likely contributed to this result. Another possible affecting factor was sample preparation. Considering the results on qPCR performance of templates produced by different methods, it is likely that reduced yields from DNA extractions introduced method-dependent error to the quantification results.

Four Åland samples showed very low concentrations of microcystins or no toxins altogether when analyzed with HPLC-DAD and LC-MS-MS methods, and yielded no signal in qPCR. The ELISA assay, however, indicated the presence of varied amounts of microcystins in these samples. Since microcystin immunoassays can be affected by the sample matrix (Metcalf et al., 2000), and the other two methods employed did not indicate high toxin concentrations, overestimated or false positive ELISA results cannot be excluded as a possibility. Microcystins are relatively stable and can persist in the water for weeks (Lahti et al., 1997), thus trace amounts could have been present in the samples, even after the producing organism no longer was.

### Conclusion

As awareness of the health risks of toxic cyanobacterial mass occurrences has grown, the demand for monitoring methods has increased. Guidelines and legislation concerning assessment of cyanobacterial risks are being brought up to date with the current scientific knowledge. A qPCR method to detect and quantify all potentially microcystin-producing members of three major bloom-forming genera, coupled to efficient sample preparation, provides a means for fast early-warning monitoring. Potentially microcystin-producing cyanobacteria can be detected with good sensitivity and a broad quantification range, responding to the significant variance in cell densities in the environment. Although in good qualitative agreement with chromatographic data, the Åland Islands gene quantification results show that small separated sample collection points are not enough to provide us with general gene copy number guidelines. Such guidelines could be established only on analysis of larger sample numbers. The Hauninen reservoir *Planktothrix* population showcases the unreliability of microscopic quantification in toxin concentration estimation, and emphasises the benefits of qPCR detection. Together all results encourage the use of molecular methods in environmental monitoring of microcystin-producing cyanobacteria.

**Table 1. Specificity of mcyB genus-specific probes and the toxins produced by the cyanobacterial strains used in this study.**

| **Cyanobacterial species** | **Strain^{a}** | **Cyanotoxins produced^{b}** | ***mcyB* amplicon detected with genus-specific probes** | | |
|---|---|---|---|---|---|
| | | | ***mcyB-*aP, *Anabaena*** | ***mcyB*-mP, *Microcystis*** | ***mcyB-*pP, *Planktothrix*** |
| *Anabaena* sp. | 90 | **MC-RR,** MC-LR, dmMC-LR, dmMC-RR | + | - | - |
| *Anabaena lemmermannii* var. *minor* | NIVA-CYA 83/1 | **dm-MC-LR**, MC-RR, dmMC-RR, MC-LR | + | - | - |
| *Anabaena lemmermannii* var. *minor* | NIVA-CYA 266/1 | **dmMC-RR, didmMC-RR,** MC-RR | + | - | - |
| *Anabaena flos-aquae* | NIVA-CYA 267/4 | **m/z 1059**, m/z 1045, dmMC-LR, MC-LR, m/z 1015, m/z 1029* | + | - | - |
| *Anabaena flos-aquae* | NIVA-CYA 499 | - | - | - | - |
| *Anabaena lapponica* | 966 | **CYN** | - | - | - |
| *Anabaena cylindrica* | PCC 73105 | - | - | - | - |
| *Anabaena cylindrica* | PCC 7938 | - | - | - | - |
| *Microcystis aeruginosa* | NIES 107 | **MC-RR,** dmMC-RR, MC-LR, dmMC-LR, MC-YR, dmMC-YR | - | + | - |
| *Microcystis aeruginosa* | NIVA-CYA 57 | **dmMC-RR,** didmMC-RR, MC-RR, MC-LR, dmMC-LR | - | + | - |
| *Microcystis aeruginosa* | NIVA-CYA 140 | MC-LR, dmMC-LR, m/z 1009 | - | + | - |
| *Microcystis aeruginosa* | NIVA-CYA 228/1 | **dmMC-RR**, didmMC-RR, MC-RR, MC-LR, dmMC-LR, MC-YR, dmMC-YR | - | + | - |
| *Microcystis aeruginosa* | NIVA-CYA 495 | **m/z 1045**, nVz 1031, MC-YR** | - | + | - |
| *Microcystis aeruginosa* | PCC 7806 | **MC-LR,** dmMC-LR | - | + | - |
| *Microcystis aeruginosa* | PCC 7820 | **MC-LR,** dmMC-LR, MC-LY, MC-LW, MC-LF | - | + | - |
| *Microcystis aeruginosa* | PCC 7941 | **MC-LR,** dmMC-LR, MC-YR | - | + | - |
| *Microcystis aeruginosa* | NIVA-CYA 22 | - | - | - | - |
| *Microcystis aeruginosa* | NIVA-CYA 464 | - | - | - | - |
| *Microcystis aeruginosa* | PCC 7005 | - | - | - | - |
| *Nodularia harveyana* | PCC 7804 | **NOD m/z 839**, NOD-R, dmNOD-R*** | - | - | - |
| *Nostoc* sp. | PCC 6310 | - | - | - | - |
| *Nostoc* sp. | PCC 7422 | - | - | - | - |
| *Planktothrix agardhii* | NIVA-CYA 15 | **didmMC-RR**, dmMC-RR | - | - | + |
| *Planktothrix agardhii* | NIVA-CYA 59/1 | **dmMC-RR**, MC-LR, dmMC-LR | - | - | + |
| *Planktothrix agardhii* | NIVA-CYA 299 | **dmMC-RR** | - | - | + |
| *Planktothrix agardhii* | PCC 7805 | - | - | - | - |
| *Planktothrix agardhii* | NIVA-CYA 12 | - | - | - | - |
| *Planktothrix agardhii* | NIVA-CYA 21 | - | - | - | - |
| *Planktothrix agardhii* | NIVA-CYA 116 | - | - | - | - |
| *Planktothrix agardhii* | PCC 7811 | - | - | - | - |

| | | | | | |
|---|---|---|---|---|---|
| "Strain names include abbreviations of culture collection names. PCC, Pasteur Culture Collection, Paris, France; NIVA-CYA, Norwegian Institute for Water Research Culture Collection, Oslo, Norway; and NIES, National Institute of Environmental Studies, Tsukuba, Japan, ^{b}Boldfacing indicates the main toxin variants, tentative identifications are asterisked: *m/z 1059 MC-HtyR, m/z 1045 MC-[ASP³] HtyR, m/z 1029 MC-FR, m/z 1015 MC-[ASP³]FR, ** m/z 1031 and m/z 1045 unknown MCs with retention times longer than MC-LR, *** m/z 839 L-HarNOD. | | | | | |

**Table 2. Sequences of primers, detection probes and quencher probes.**

| Oligonucleotide | 5'-3' sequence | Tₘ/°C | Oligonucleotide type | Specificity |
|---|---|---|---|---|
| mcyBHF03A | GCTTTAATCCACAAGAAGCTTTATTAGC | 56,1 | forward primer | *Anabaena* |
| mcyBHF03M | AGATTTTAATCCACAAGAAGCTTTATTAGC | 56,1 | forward primer | *Microcystis* |
| mcyBHF03P | GGTTTAATCAACAAGAGGCTTTATTAGC | 55,9 | forward primer | *Planktothrix* |
| mcyBHR04 | CTGTTGCCTCCTAGTTCAAAAAATGACT | 58,5 | reverse primer | All three target genera |
| mcyB-aP | ACTGAATTATTGGAGGTAGAGGTGAGTGATAC | 58,9 | detection probe | *Anabaena* |
| mcyB-aQ | CCTCTACCTCCAATAATTCA | 43,6 | quencher probe | *Anabaena* |
| mcyB-mP | GGGTGAGTTATTAGAAGCAGAAGTTAGTAACAG | 58,4 | detection probe | *Microcystis* |
| mcyB-mQ | TTCTGCTTCTAATAACTCACC | 43,4 | quencher probe | *Microcystis* |
| mcyB-pP | GGGGTGAATTATTAGAAATAGAAGTAAGTGACAA | 59,2 | detection probe | *Planktothrix* |
| mcyB-pQ | TTACTTCTATTTCTAATAATTCACC | 43,9 | quencher probe | *Planktothrix* |

**Table 3. Genus-specific mcyB copy numbers, microcystin amounts and identified microcystin variants in 13 lakes on Åland Islands during the time period from July 14^{th} to July 16^{th} 2008.**

| Lake | *mcyB* copies / g sample dry weight | | | Microcystin concentrations in MC-LR equivalents, µg total MC / g sample dry weight | | | |
|---|---|---|---|---|---|---|---|
| | *Anabaena* | *Microcystis* | *Planktothrix* | HPLC-DAD | ELISA | LC-MS-MS | Microcystin variants identified by LC-MS-MS |
| Godby träsk | - | 1,0E+08 | - | 40.4 | 62.2 | 24.5 | dmMC-RR, MC-RR, MC-YR, dmMC-LR, MC-LR |
| Brantsbole träsk | 4,1E+07 | 2,0E+08 | - | 43.0 | 94.0 | 95.3 | MC-YR, dmMC-LR, MC-LR, MC-LY,MC-LW, MC-LF |
| Gloskärs träsk | - | - | - | 0 | 6.6 | 0.67 | dmMC-LR |
| Tjudö träsk | - | 1,4E+09 | - | 67.9 | 49.5 | 45.7 | dmMC-RR, MC-RR, MC-dmMC-LR, MC-LR, MC-LA |
| Markusbölefjärden | - | - | - | 0 | 1.2 | 0.37 | MC-LR |
| Nåtö hemviken | - | 3,9E+11 | - | 114.8 | 137.8 | 85.6 | dmMC-RR, MC-RR, MC-YR, dmMC-LR, MC-LR |
| Hammarlands | - | 1,3E+09 | - | 0.97 | 1.5 | 1.65 | MC-LR |
| långträsk Lemböte byträsk | - | 2,8E+10 | - | 0 | 3.4 | 2.5 | MC-YR, dmMC-LR, MC-LR |
| Vargata träsk | - | 1,3E+11 | - | 78.3 | 145.1 | 71.3 | dmMC-RR, MC-RR, MC-YR, dmMC-LR, MC-LR |
| Kaldersfjärden | - | 1,3E+09 | 3,3E+08 | 0 | 8.3 | 11.8 | MC-YR, dmMC-LR, MC-LR |
| Norsträsk | - | 4,0E+07 | - | 0 | 6.1 | 1.4 | dmMC-LR, MC-LR |
| 1,*ang,sjiin | - | - | - | 0 | 0.1 | 0 | - |
| Slussfjärden | - | - | - | 0 | 0.9 | 0 | - |

### REFERENCES

Al-Tebrineh, J., Gehringer, M.M., Akcaalan, R., Neilan, B.A., 2011. A new quantitative PCR assay for the detection of hepatotoxigenic cyanobacteria. Toxicon 57, 546-554.
Becker, S., Fahrbach, M., Boger, P., Ernst, A., 2002. Quantitative tracing, by Taq nuclease assays, of a synechococcus ecotype in a highly diversified natural population. Appl. Environ. Microbiol. 68, 4486-4494.
Briand, E., Gugger, M., Francois, J.C., Bernard, C., Humbert, J.F., Quiblier, C., 2008. Temporal variations in the dynamics of potentially microcystin-producing strains in a bloom-forming Planktothrix agardhii (cyanobacteria) population. Appl. Environ. Microbiol. 74, 3839-3848.
Christiansen, G., Fastner, J., Erhard, M., Börner, T., Dittmann, E., 2003. Microcystin biosynthesis in Planktothrix: genes, evolution, and manipulation. J. Bacteriol. 185, 564-572.
Fastner, J., Erhard, M., Carmichael, W.W., Sun, F., Rinehart, K.L., Ronicke, H., Chorus, I., 1999. Characterization and diversity of microcystins in natural blooms and strains of the genera Microcystis and Planktothrix from German freshwaters. Arch. Hydrobiol. 45, 147-163.
Fiore, M.F., Genuario, D.B., da Silva, C.S., Shishido, T.K., Moraes, L.A., Cantusio Neto, R., Silva-Stenico, M.E., 2009. Microcystin production by a freshwater spring cyanobacterium of the genus Fischerella. Toxicon 53, 754-761.
Fortin, N., Aranda-Rodriguez, R., Jing, H., Pick, F., Bird, D., Greer, C.W., 2010. Detection of microcystin-producing cyanobacteria in Missisquoi Bay, Quebec, Canada, using quantitative PCR. Appl. Environ. Microbiol. 76, 5105-5112.
Foulds, I.V., Granacki, A., Xiao, C., Krull, U., Castle, A., Horgen, P.A., 2002. Quantification of microcystin-producing cyanobacteria and E. coli in water by 5'-nuclease PCR. J. Appl. Microbiol. 93, 825-834.
Furukawa, K., Noda, N., Tsuneda, S., Saito, T., Itayama, T., Inamori, Y., 2006. Highly sensitive real-time PCR assay for quantification of toxic cyanobacteria based on microcystin synthetase A gene. J Biosci Bioeng 102, 90-96.
Hisbergues, M., Christiansen, G., Rouhiainen, L., Sivonen, K., Börner, T., 2003. PCR-based identification of microcystin-producing genotypes of different cyanobacterial genera. Arch. Microbiol. 180, 402-410.
Izaguirre, G., Jungblut, A.D., Neilan, B.A., 2007. Benthic cyanobacteria (Oscillatoriaceae) that produce microcystin-LR, isolated from four reservoirs in southern California. Water Res. 41, 492-498.
Jochimsen, E.M., Carmichael, W.W., An, J.S., Cardo, D.M., Cookson, S.T., Holmes, C.E., Antunes, M.B., de Melo Filho, D.A., Lyra, T.M., Barreto, V.S., Azevedo, S.M., Jarvis, W.R., 1998. Liver failure and death after exposure to microcystins at a hemodialysis center in Brazil. N. Engl. J. Med. 338, 873-878.
Kaebernick, M., Dittmann, E., Börner, T., Neilan, B.A., 2002. Multiple alternate transcripts direct the biosynthesis of microcystin, a cyanobacterial nonribosomal peptide. Appl. Environ. Microbiol. 68, 449-455.
Kaebernick, M., Neilan, B.A., Börner, T., Dittmann, E., 2000. Light and the transcriptional response of the microcystin biosynthesis gene cluster. Appl. Environ. Microbiol. 66, 3387-3392.
Kaneko, T., Nakajima, N., Okamoto, S., Suzuki, I., Tanabe, Y., Tamaoki, M., Nakamura, Y., Kasai, F., Watanabe, A., Kawashima, K., Kishida, Y., Ono, A., Shimizu, Y., Takahashi, C., Minami, C., Fujishiro, T., Kohara, M., Katoh, M., Nakazaki, N., Nakayama, S., Yamada, M., Tabata, S., Watanabe, M.M., 2007. Complete genomic structure of the bloom-forming toxic cyanobacterium Microcystis aeruginosa NIES-843. DNA Res. 14, 247-256.
Kurmayer, R., Christiansen, G., Fastner, J., Börner, T., 2004. Abundance of active and inactive microcystin genotypes in populations of the toxic cyanobacterium Planktothrix spp. Environ. Microbiol. 6, 831-841.
Kurmayer, R., Kutzenberger, T., 2003. Application of real-time PCR for quantification of microcystin genotypes in a population of the toxic cyanobacterium Microcystis sp. Appl. Environ. Microbiol. 69, 6723-6730.
Labarre, J., Chauvat, F., Thuriaux, P., 1989. Insertional mutagenesis by random cloning of antibiotic resistance genes into the genome of the cyanobacterium Synechocystis strain PCC 6803. J. Bacteriol. 171, 3449-3457.
Lahti, K., Rapala, J., Färdig, M., Niemelä, M., Sivonen, K., 1997. Persistence of cyanobacterial hepatotoxin, microcystin-LR in particulate material and dissolved in lake water. Water Res. 31, 1005-1012.
Mbedi, S., Welker, M., Fastner, J., Wiedner, C., 2005. Variability of the microcystin synthetase gene cluster in the genus Planktothrix (Oscillatoriales, Cyanobacteria). FEMS Microbiol. Lett. 245, 299-306.
Metcalf, J.S., Hyenstrand, P., Beattie, K.A., Codd, G.A., 2000. Effects of physicochemical variables and cyanobacterial extracts on the immunoassay of microcystin-LR by two ELISA kits. J. Appl. Microbiol. 89, 532-538.
Mikalsen, B., Boison, G., Skulberg, O.M., Fastner, J., Davies, W., Gabrielsen, T.M., Rudi, K., Jakobsen, K.S., 2003. Natural variation in the microcystin synthetase operon mcyABC and impact on microcystin production in Microcystis strains. J. Bacteriol. 185, 2774-2785.
Moffitt, M.C., Neilan, B.A., 2004. Characterization of the nodularin synthetase gene cluster and proposed theory of the evolution of cyanobacterial hepatotoxins. Appl. Environ. Microbiol. 70, 6353-6362.
Nishizawa, T., Asayama, M., Fujii, K., Harada, K., Shirai, M., 1999. Genetic analysis of the peptide synthetase genes for a cyclic heptapeptide microcystin in Microcystis spp. J Biochem 126, 520-529.
Nonnemann, D., Zimba, P.V., 2002. A PCR-based test to assess the potential for microcystin occurrence in channel catfish production ponds. J. Phycol. 38, 230-233.
Nurmi, J., Wikman, T., Karp, M., Lövgren, T., 2002. High-performance real-time quantitative RT-PCR using lanthanide probes and a dual-temperature hybridization assay. Anal. Chem. 74, 3525-3532.
Ostermaier, V., Kurmayer, R., 2009. Distribution and Abundance of Nontoxic Mutants of Cyanobacteria in Lakes of the Alps. Microb. Ecol. 58, 323-333.
Ouahid, Y., Perez-Silva, G., del Campo, F.F., 2005. Identification of potentially toxic environmental Microcystis by individual and multiple PCR amplification of specific microcystin synthetase gene regions. Environ. Toxicol. 20, 235-242.
Pilotto, L.S., Douglas, R.M., Burch, M.D., Cameron, S., Beers, M., Rouch, G.J., Robinson, P., Kirk, M., Cowie, C.T., Hardiman, S., Moore, C., Attewell, R.G., 1997. Health effects of exposure to cyanobacteria (blue-green algae) during recreational water-related activities. Aust. N. Zealand J. Public Health 21, 562-566.
Prinsep, M., Caplan, F., Moore, R., Patterson, G., Honkanen, R., Boynton, A., 1992. Microcystin-LA from a blue-green alga belonging to the stigonematales. Phytochemistry 31, 1247-1248.
Rasmussen, J.P., Barbez, P.H., Burgoyne, L.A., Saint, C.P., 2008. Rapid preparation of cyanobacterial DNA for real-time PCR analysis. Lett. Appl. Microbiol. 46, 14-19.
Rinta-Kanto, J.M., Ouellette, A.J., Boyer, G.L., Twiss, M.R., Bridgeman, T.B., Wilhelm, S.W., 2005. Quantification of toxic Microcystis spp. during the 2003 and 2004 blooms in western Lake Erie using quantitative real-time PCR. Environ. Sci. Technol. 39, 4198-4205.
Rouhiainen, L., Vakkilainen, T., Siemer, B.L., Buikema, W., Haselkorn, R., Sivonen, K., 2004. Genes coding for hepatotoxic heptapeptides (microcystins) in the cyanobacterium Anabaena strain 90. Appl. Environ. Microbiol. 70, 686-692.
Schober, E., Kurmayer, R., 2006. Evaluation of different DNA sampling techniques for the application of the real-time PCR method for the quantification of cyanobacteria in water. Lett. Appl. Microbiol. 42, 412-417.
Sevilla, E., Martin-Luna, B., Vela, L., Bes, M.T., Fillat, M.F., Peleato, M.L., 2008. Iron availability affects mcyD expression and microcystin-LR synthesis in Microcystis aeruginosa PCC7806. Environ. Microbiol. 10, 2476-2483.
Sivonen, K., 2008. Emerging high throughput analyses of cyanobacterial toxins and toxic cyanobacteria. Adv. Exp. Med. Biol. 619, 539-557.
Sivonen, K., Jones, G., 1999. Cyanobacterial toxins, in: Bartram, J., Chorus, I. (Eds.). Spon Press, UK, pp. 41-111.
Soini, E., Lövgren, T., 1987. Time-Resolved Fluorescence of Lanthanide Probed and Applications in Biotechnology. Crit. Rev. Anal. Chem. 18, 105-154.
Spoof, L., Meriluoto, J., 2005. Analysis of microcystins by high-performance liquid chromatography with photodiode-array detection., in: Meriluoto, J., Codd, G.A. (Eds.). Åbo Akademi University Press, Turku, Finland, pp. 77-84.
Spoof, L., Vesterkvist, P., Lindholm, T., Meriluoto, J., 2003. Screening for cyanobacterial hepatotoxins, microcystins and nodularin in environmental water samples by reversed-phase liquid chromatography-electrospray ionisation mass spectrometry. J. Chromatogr. A 1020, 105-119.
Staub, R., 1961. Ernährungsphysiologisch-autökologische Untersuchungen an der planktischen Blaualge Oscillatoria rubescens DC. Schweitz. Z. Hydrol. 23, 82-198.
Stewart, I., Seawright, A.A., Shaw, G.R., 2008. Cyanobacterial poisoning in livestock, wild mammals and birds--an overview. Adv. Exp. Med. Biol. 619, 613-637.
Svircev, Z., Krstic, S., Miladinov-Mikov, M., Baltic, V., Vidovic, M., 2009. Freshwater cyanobacterial blooms and primary liver cancer epidemiological studies in Serbia. J. Environ. Sci. Health. C. Environ. Carcinog. Ecotoxicol. Rev. 27, 36-55.
Tillett, D., Dittmann, E., Erhard, M., von Döhren, H., Börner, T., Neilan, B.A., 2000. Structural organization of microcystin biosynthesis in Microcystis aeruginosa PCC7806: an integrated peptide-polyketide synthetase system. Chem. Biol. 7, 753-764.
Turner, P.C., Gammie, A.J., Hollinrake, K., Codd, G.A., 1990. Pneumonia associated with contact with cyanobacteria. BMJ 300, 1440-1441.
Ueno, Y., Nagata, S., Tsutsumi, T., Hasegawa, A., Watanabe, M.F., Park, H.D., Chen, G.C., Chen, G., Yu, S.Z., 1996. Detection of microcystins, a blue-green algal hepatotoxin, in drinking water sampled in Haimen and Fusui, endemic areas of primary liver cancer in China, by highly sensitive immunoassay. Carcinogenesis 17, 1317-1321.
Vaitomaa, J., Rantala, A., Halinen, K., Rouhiainen, L., Tallberg, P., Mokelke, L., Sivonen, K., 2003. Quantitative real-time PCR for determination of microcystin synthetase E copy numbers for Microcystis and Anabaena in lakes. Appl. Environ. Microbiol. 69, 7289-7297.
Vezie, C., Brient, L., Sivonen, K., Bertru, G., Lefeuvre, J., Salkinoja-Salonen, M., 1998. Variation of Microcystin Content of Cyanobacterial Blooms and Isolated Strains in Lake Grand-Lieu (France). Microb. Ecol. 35, 126-135.
Welker, M., von Döhren, H., 2006. Cyanobacterial peptides - nature's own combinatorial biosynthesis. FEMS Microbiol. Rev. 30, 530-563.
Wilson, I.G., 1997. Inhibition and facilitation of nucleic acid amplification. Appl. Environ. Microbiol. 63, 3741-3751.
Yu, S.Z., 1995. Primary prevention of hepatocellular carcinoma. J. Gastroenterol. Hepatol. 10, 674-682.

## Claims

1. Method for detecting the presence of microcystin-producing toxic cyanobacteria in a sample comprising the steps of:
a) performing lysis of the cells in said sample;
b) contacting nucleic acid obtained from the lysed cells in a polymerase chain reaction mix with primers hybridizing with the nucleic acid sequence of SEQ ID NO:1, SEQ ID NO:2 and SEQ ID NO:3 in the *mcyB* gene present in *Microcystis aeruginosa*, *Planktothrix agardhii* and *Anabaena sp*., wherein said primers amplify at least part of said sequences in the *mcyB* gene;
c) performing a polymerase chain reaction with a reaction mix obtained from step b) so that the sequences of said *mcyB* gene are amplified, if said sequences are present in the sample; and
d) detecting the presence of amplified nucleid acid sequences of said mcyB gene, wherein the presence of amplified *mcyB* gene sequence is indicative of the presence of microcystin-producing toxic cyanobacteria in the sample.

2. The method according to claim 1, wherein at least one of the primes comprises one of the following sequences:
5'-GCTTTAATCCACAAGAAGCTTTATTAGC-3' (SEQ ID NO:4)
5'-AGATTTTAATCCACAAGAAGCTTTATTAGC-3' (SEQ ID NO:5)
5'-GGTTTAATCAACAAGAGGCTTTATTAGC-3' (SEQ ID NO:6)
5'-CTGTTGCCTCCTAGTTCAAAAAATGACT-3' (SEQ ID NO:7)

3. The method according to claim 1 or 2, wherein said polymerase chain reaction mix is for real-time polymerase chain reaction.

4. The method according to claim 3, wherein at least one of the following probes is used in the reaction:
5'-ACTGAATTATTGGAGGTAGAGGTGAGTGATAC-3' (SEQ ID NO:8)
5'-CCTCTACCTCCAATAATTCA-3' (SEQ ID NO:9)
5'- GGGTGAGTTATTAGAAGCAGAAGTTAGTAACAG-3' (SEQ ID NO:10)
5'-TTCTGCTTCTAATAACTCACC-3' (SEQ ID NO:11)
5'-GGGGTGAATTATTAGAAATAGAAGTAAGTGACAA-3' (SEQ ID NO:12)
5'-TTACTTCTATTTCTAATAATTCACC-3' (SEQ ID NO:13)

5. The method according to claim 3 or 4, wherein the real-time polymerase chain reaction is a quantitative-PCR reaction and the method comprises a further step of determining the gene copy number of the *mcyB* gene in said sample.

6. The method according to claim 5 comprising a further step of monitoring the toxic microcystin concentration in the sample by correlating said gene copy number obtained by quantitative-PCR to the corresponding microcystin concentration.

7. A kit for the detection of the presence of microcystin-producing toxic cyanobacteria in a sample comprising a set of oligonucleotide primers as set forth in SEQ ID NOS:4-7 and any of the oligonucleotide probes as set forth in SEQ ID NOS:8-13.

8. The kit according to claim 7, wherein said microcystin-producing toxic cyanobacteria is selected from the group consisting of *Microcystis aeruginosa*, *Planktothrix agardhii* and *Anabaena sp.*

9. Use of a kit according to claim 7 or 8 for the detection of the presence of microcystin-producing toxic cyanobacteria in a sample.

## Patentansprüche

1. Verfahren zum Detektieren des Vorhandenseins von Microcystin produzierenden toxischen Cyanobakterien in einer Probe, welches die folgenden Schritte umfasst:
a) Durchführen einer Lyse der Zellen in der Probe;
b) Inkontaktbringen einer aus den lysierten Zellen erhaltenen Nukleinsäure in einem Polymerasekettenreaktionsgemisch mit Primern, die mit der Nukleinsäuresequenz von SEQ ID NO: 1, SEQ ID NO: 2 und SEQ ID NO: 3 in dem in *Microcystis aeruginosa, Planktothrix agardhii* und *Anabaena sp.* vorhandenen *mcyB*-Gen hybridisieren, wobei die Primer mindestens einen Teil der Sequenzen in dem *mcyB*-Gen amplifizieren;
c) Durchführen einer Polymerasekettenreaktion mit einem aus Schritt b) erhaltenen Reaktionsgemisch, so dass die Sequenzen des *mcyB*-Gens amplifiziert werden, wenn die Sequenzen in der Probe vorhanden sind; und
d) Detektieren des Vorhandenseins von amplifizierten Nukleinsäuresequenzen des mcyB-Gens, wobei das Vorhandensein einer amplifizierten *mcyB*-Gensequenz auf das Vorhandensein von Microcystin produzierenden toxischen Cyanobakterien in der Probe hinweist.

2. Verfahren nach Anspruch 1, wobei mindestens einer der Primes eine der folgenden Sequenzen umfasst:
5'-GCTTTAATCCACAAGAAGCTTTATTAGC-3' (SEQ ID NO:4)
5'-AGATTTTAATCCACAAGAAGCTTTATTAGC-3' (SEQ ID NO:5)
5'-GGTTTAATCAACAAGAGGCTTTATTAGC-3' (SEQ ID NO:6)
5'-CTGTTGCCTCCTAGTTCAAAAAATGACT-3' (SEQ ID NO:7)

3. Verfahren nach Anspruch 1 oder 2, wobei das Polymerasekettenreaktionsgemisch für eine Echtzeit-Polymerasekettenreaktion bestimmt ist.

4. Verfahren nach Anspruch 3, wobei mindestens eine der folgenden Sonden in der Reaktion verwendet wird:
5'-ACTGAATTATTGGAGGTAGAGGTGAGTGATAC-3' (SEQ ID NO:8)
5'-CCTCTACCTCCAATAATTCA-3' (SEQ ID NO:9)
5'- GGGTGAGTTATTAGAAGCAGAAGTTAGTAACAG-3' (SEQ ID NO:10)
5'-TTCTGCTTCTAATAACTCACC-3' (SEQ ID NO:11)
5'-GGGGTGAATTATTAGAAATAGAAGTAAGTGACAA-3' (SEQ ID NO:12)
5'-TTACTTCTATTTCTAATAATTCACC-3' (SEQ ID NO:13)

5. Verfahren nach Anspruch 3 oder 4, wobei die Echtzeit-Polymerasekettenreaktion eine quantitative PCR-Reaktion ist und das Verfahren einen weiteren Schritt des Bestimmens der Genkopienzahl des mcyB-Gens in der Probe umfasst.

6. Verfahren nach Anspruch 5, umfassend einen weiteren Schritt des Überwachens der Konzentration an toxischem Microcystin in der Probe durch Korrelieren der durch quantitative PCR erhaltenen Genkopienzahl mit der entsprechenden Microcystinkonzentration.

7. Kit zur Detektion des Vorhandenseins von Microcystin produzierenden toxischen Cyanobakterien in einer Probe, umfassend einen Satz von Oligonukleotidprimern wie in SEQ ID NO: 4-7 ausgeführt und eine beliebige der Oligonukleotidsonden wie in SEQ ID NO: 8-13 ausgeführt.

8. Kit nach Anspruch 7, wobei die Microcystin produzierenden toxischen Cyanobakterien aus der Gruppe bestehend aus *Microcystis aeruginosa*, *Planktothrix agardhii* und *Anabaena sp.* ausgewählt ist.

9. Verwendung eines Kits nach Anspruch 7 oder 8 zur Detektion des Vorhandenseins von Microcystin produzierenden toxischen Cyanobakterien in einer Probe.

## Revendications

1. Procédé pour détecter la présence de cyanobactéries toxiques produisant de la microcystine dans un échantillon comprenant les étapes suivantes :
a) effectuer une lyse des cellules dans ledit échantillon ;
b) mettre en contact l'acide nucléique obtenu à partir des cellules lysées dans un mélange de réaction en chaîne par polymérase avec des amorces s'hybridant avec la séquence d'acides nucléiques SEQ ID NO : 1, SEQ ID NO : 2, et SEQ ID NO : 3 dans le gène *mcyB* présent dans *Microcystis aeruginosa, Planktothrix agardhii* et *Anabaena sp.,* lesdites amorces amplifiant au moins une partie desdites séquences dans le gène *mcyB* ;
c) effectuer une réaction en chaîne par polymérase avec un mélange réactionnel obtenu à partir de l'étape b) de sorte que les séquences dudit gène *mcyB* sont amplifiées, si lesdites séquences sont présentes dans l'échantillon ; et
d) détecter la présence de séquences d'acides nucléiques amplifiées dudit gène mcyB, la présence de séquence du gène *mcyB* amplifiée étant un indicateur de la présence de cyanobactéries toxiques produisant de la microcystine dans l'échantillon.

2. Procédé selon la revendication 1, dans lequel au moins l'une des primes comprend l'une des séquences suivantes :
5'-GCTTTAATCCACAAGAAGCTTTATTAGC-3' (SEQ ID NO : 4)
5'-AGATTTTAATCCACAAGAAGCTTTATTAGC-3' (SEQ ID NO : 5)
5'-GGTTTAATCAACAAGAGGCTTTATTAGC-3' (SEQ ID NO : 6)
5'-CTGTTGCCTCCTAGTTCAAAAAATGACT-3' (SEQ ID NO : 7)

3. Procédé selon la revendication 1 ou 2, dans lequel ledit mélange de réaction en chaîne par polymérase est destiné à une réaction en chaîne par polymérase en temps réel.

4. Procédé selon la revendication 3, dans lequel au moins l'une des sondes suivantes est utilisée dans la réaction :
5'-ACTGAATTATTGGAGGTAGAGGTGAGTGATAC-3' (SEQ ID NO : 8)
5'-CCTCTACCTCCAATAATTCA-3' (SEQ ID NO : 9)
5'-GGGTGAGTTATTAGAAGCAGAAGTTAGTAACAG-3' (SEQ ID NO : 10)
5'-TTCTGCTTCTAATAACTCACC-3' (SEQ ID NO : 11)
5'-GGGGTGAATTATTAGAAATAGAAGTAAGTGACAA-3' (SEQ ID NO : 12)
5'-TTACTTCTATTTCTAATAATTCACC-3' (SEQ ID NO : 13)

5. Procédé selon la revendication 3 ou 4, dans lequel la réaction en chaîne par polymérase en temps réel est une réaction PCR quantitative et le procédé comprend une étape supplémentaire consistant à déterminer le nombre de copies de gène du gène *mcyB* dans ledit échantillon.

6. Procédé selon la revendication 5 comprenant une étape supplémentaire consistant à surveiller la concentration en microcystine toxique dans l'échantillon par corrélation dudit nombre de copies de gène obtenu par PCR quantitative à la concentration en microcystine correspondante.

7. Kit pour la détection de la présence de cyanobactéries toxiques produisant de la microcystine dans un échantillon comprenant un ensemble d'amorces oligonucléotidiques telles qu'indiquées dans les séquences SEQ ID NO: 4 à 7 et l'une quelconque des sondes nucléotidique telles qu'indiquées dans les SEQ ID NO : 8 à 13.

8. Kit selon la revendication 7, dans lequel lesdites cyanobactéries toxiques produisant de la microcystine est choisie dans le groupe constitué par *Microcystis aeruginosa, Planktothrix agardhii* et *Anabaena sp.*

9. Utilisation d'un kit selon la revendication 7 ou 8 pour la détection de la présence de cyanobactéries toxiques produisant de la microcystine dans un échantillon.


### SEQUENCE LISTING

<110> Turun yliopisto
<120> Method and primers for the detection of microcystin-producing toxic
   cyanobacteria
<160> 20
<170> BiSSAP 1.0
<210> 1
   <211> 105
   <212> DNA
   <213> Microcystis aeruginosa UV027
<220>
   <221> SOURCE
   <222> 1..105
   <223> /mol_type="DNA" /organism="Microcystis aeruginosa UV027"
<400> 1
   agattttaat ccacaagaag ctttattagc ttccctttgg ggtgagttat tagaagcaga 60
   agttagtaac agtaatcagt cattttttga actaggagga aacag 105
<210> 2
   <211> 103
   <212> DNA
   <213> Planktothrix agardhii NIVA-CYA 126/8
<220>
   <221> source
   <222> 1..103
   <223> /mol_type="DNA" /note="A PCR primer" /organism="Planktothrix agardhii NIVA-CYA 126/8"
<400> 2
   ggtttaatca acaagaggct ttattagcct tggtttgggg tgaattatta gaaatagaag 60
   taagtgacaa taatcagtca ttttttgaac tcggaggcaa tag 103
<210> 3
   <211> 103
   <212> DNA
   <213> Anabaena sp.
<220>
   <221> SOURCE
   <222> 1..103
   <223> /mol_type="DNA" /organism="Anabaena sp."
<400> 3
   gctttaatcc acaagaagct ttattagcct ccatttggac tgaattattg gaggtagagg 60
   tgagtgatac taatcagtca ttttttgagc taggaggaaa tag 103
<210> 4
   <211> 28
   <212> DNA
   <213> Anabaena sp.
<220>
   <221> SOURCE
   <222> 1..28
   <223> /mol_type="DNA" /organism="Anabaena sp."
<400> 4
   gctttaatcc acaagaagct ttattagc 28
<210> 5
   <211> 30
   <212> DNA
   <213> Microcystis aeruginosa UV027
<220>
   <221> SOURCE
   <222> 1..30
   <223> /mol_type="DNA" /organism="Microcystis aeruginosa UV027"
<400> 5
   agattttaat ccacaagaag ctttattagc 30
<210> 6
   <211> 28
   <212> DNA
   <213> Planktothrix agardhii NIVA-CYA 126/8
<220>
   <221> SOURCE
   <222> 1..28
   <223> /mol_type="DNA" /organism="Planktothrix agardhii NIVA-CYA 126/8"
<400> 6
   ggtttaatca acaagaggct ttattagc 28
<210> 7
   <211> 28
   <212> DNA
   <213> Planktothrix agardhii NIVA-CYA 126/8
<220>
   <221> SOURCE
   <222> 1..28
   <223> /mol_type="DNA" /organism="Planktothrix agardhii NIVA-CYA 126/8"
<400> 7
   ctgttgcctc ctagttcaaa aaatgact 28
<210> 8
   <211> 32
   <212> DNA
   <213> Anabaena sp.
<220>
   <221> source
   <222> 1..32
   <223> /mol_type="DNA" /note="A PCR primer" /organism="Anabaena sp."
<400> 8
   actgaattat tggaggtaga ggtgagtgat ac 32
<210> 9
   <211> 20
   <212> DNA
   <213> Anabaena sp.
<220>
   <221> SOURCE
   <222> 1..20
   <223> /mol_type="DNA" /organism="Anabaena sp."
<400> 9
   cctctacctc caataattca 20
<210> 10
   <211> 33
   <212> DNA
   <213> Microcystis aeruginosa UV027
<220>
   <221> SOURCE
   <222> 1..33
   <223> /mol_type="DNA" /organism="Microcystis aeruginosa UV027"
<400> 10
   gggtgagtta ttagaagcag aagttagtaa cag 33
<210> 11
   <211> 21
   <212> DNA
   <213> Microcystis aeruginosa UV027
<220>
   <221> source
   <222> 1..21
   <223> /mol_type="DNA" /note="A PCR primer" /organism="Microcystis aeruginosa UV027"
<400> 11
   ttctgcttct aataactcac c 21
<210> 12
   <211> 34
   <212> DNA
   <213> Planktothrix agardhii NIVA-CYA 126/8
<220>
   <221> source
   <222> 1..34
   <223> /mol_type="DNA" /note="A PCR primer" /organism="Planktothrix agardhii NIVA-CYA 126/8"
<400> 12
   ggggtgaatt attagaaata gaagtaagtg acaa 34
<210> 13
   <211> 25
   <212> DNA
   <213> Planktothrix agardhii NIVA-CYA 126/8
<220>
   <221> source
   <222> 1..25
   <223> /mol_type="DNA" /note="A PCR primer" /organism="Planktothrix agardhii NIVA-CYA 126/8"
<400> 13
   ttacttctat ttctaataat tcacc 25
<210> 14
   <211> 120
   <212> DNA
   <213> Anabaena sp.
<220>
   <221> source
   <222> 1..120
   <223> /mol_type="DNA" /organism="Anabaena sp."
<400> 14
   caaattacta gctttaatcc acaagaagct ttattagcct ccatttggac tgaattattg     60
   gaggtagagg tgagtgatac taatcagtca ttttttgagc taggaggaaa tagtttaaag    120
<210> 15
   <211> 120
   <212> DNA
   <213> Microcystis aeruginosa
<220>
   <221> source
   <222> 1..120
   <223> /mol_type="DNA" /organism="Microcystis aeruginosa"
<400> 15
   caagtcccag attttaatcc acaagaagct ttattagctt ccctttgggg tgagttatta     60
   gaagcagaag ttagtaacag taatcagtca ttttttgaac taggaggaaa cagtctgaag    120
<210> 16
   <211> 120
   <212> DNA
   <213> Planktothrix agardhii
<220>
   <221> source
   <222> 1..120
   <223> /mol_type="DNA" /organism="Planktothrix agardhii"
<400> 16
   caaccaactg ggtttaatca acaagaggct ttattagcct tggtttgggg tgaattatta     60
   gaaatagaag taagtgacaa taatcagtca ttttttgaac tcggaggcaa tagtttaaaa    120
<210> 17
   <211> 28
   <212> DNA
   <213> Microcystis aeruginosa
<220>
   <221> source
   <222> 1..28
   <223> /mol_type="DNA" /organism="Microcystis aeruginosa"
<400> 17
   agtcattttt tgaactagga ggcaacag 28
<210> 18
   <211> 20
   <212> DNA
   <213> Anabaena <cyanobacteria>
<220>
   <221> source
   <222> 1..20
   <223> /mol_type="DNA" /organism="Anabaena <cyanobacteria>"
<400> 18
   tgaattattg gaggtagagg 20
<210> 19
   <211> 21
   <212> DNA
   <213> Microcystis aeruginosa
<220>
   <221> source
   <222> 1..21
   <223> /mol_type="DNA" /organism="Microcystis aeruginosa"
<400> 19
   ggtgagttat tagaagcaga a 21
<210> 20
   <211> 25
   <212> DNA
   <213> Planktothrix agardhii
<220>
   <221> source
   <222> 1..25
   <223> /mol_type="DNA" /organism="Planktothrix agardhii"
<400> 20
   ggtgaattat tagaaataga agtaa 25